# FASCICULE DE BREVET EUROPEEN

(11) **EP 0 323 762 B1**
(45) Date de publication et mention de la délivrance du brevet: **14.07.1993**
(21) Numéro de dépôt: 88400018.3
(22) Date de dépôt: 06.01.1988
(51) Int. Cl.: A61K 31/485, A61K 9/08, A61K 9/10

(54) **Nouvelles compositions anti-tussives et leur procédé d'obtention**
Hustenstillende Zusammensetzungen und ein Verfahren zu deren Herstellung
Antitussive compositions and their preparation

(43) Date de publication de la demande: 12.07.1989
(73) Titulaire: BOUCHARA S.A., F-75003 Paris (FR)
(72) Inventeur: Streichenberger, Gilles, FR-60270 GOUVIEUX (FR)
(74) Mandataire: Burtin, Jean-François Bouchara S.A.

(56) Documents cités:
- FR-A- 2 601 246
- US-A- 4 454 140

## Description

La présente invention se rapporte à de nouvelles compositions pharmaceutiques manifestant des propriétés anti-tussives.

Elle a plus particulièrement pour objet des compositions pharmaceutiques à base de Dextrométhorphane ou d'un de ses sels.

Elle a spécifiquement pour objet de nouvelles compositions pharmaceutiques destinées à l'usage transmuqueux à base de Dextrométhorphane ou d'un de ses sels d'addition avec un acide minéral ou organique.

On sait, depuis près de 10 ans (Brevet français 1.371.454) qu'il est possible d'administrer certains principes actifs par voie nasale comme par exemple des broncholytiques, des alcaloïdes de l'ergot de seigle ou des hormones peptidiques et qu'il en résulte une absorption systémique.

Cette voie d'administration présente donc l'avantage par rapport à la voie d'administration digestive, qu'après avoir traversé la muqueuse nasale le principe actif passe directement dans la circulation sanguine. Il en résulte une biodisponibilité immédiate du principe actif et un début rapide d'action thérapeutique.

Ce principe n'a jusqu'ici pas été élargi et il semble exclu qu'il puisse être utilisé pour tout type de médicament car rien ne permet d'établir, à priori, un passage par voie pernasale. En effet, rien ne permet de prévoir si un principe actif sera susceptible de passer intact à travers la muqueuse nasale et d'assurer une concentration sanguine efficace. Un grand nombre de principes actifs ne sont actifs par cette voie qu'à des concentrations élevées. Il s'y ajoute des problèmes de tolérence locale ou de possibilité d'irritation si le pH de la solution de la suspension à administrer se situe en dehors des limites physiologiques.

En outre, il a été constaté (Brevet français 2.539.994) que certaines substances comme les ergolines administrées par voie nasale inhibaient d'une façon irréversible la fonction ciliaire.

On connaissait également par le Brevet américain 4.454.140 (Hoffmann-Laroche) la possibilité d'administrer le Dextrométhorphane par voie pernasale mais les informations contenues dans ce brevet montrent qu'aux doses élevées utilisées et avec la formulation employée, le passage systémique est très variable selon les sujets et reste très inférieur à celui de l'administration intraveineuse.

Or, il vient d'être trouvé - et c'est sur cette constatation expérimentale que repose la présente invention - que le Dextrométhorphane ou un de ses sels d'addition avec un acide minéral ou organique administré par voie transmuqueuse manifestait une efficacité élevée tout en pouvant être administré à des concentrations très faibles, même très inférieures à celles qui sont utilisées par voie orale. A ces concentrations, en outre le Dextrométhorphane ne modifie pas le rythme des battements ciliaires.

Le Dextrométhorphane ((+) 3- méthoxy N-méthyl morphinane)et ses sels, notamment le bromhydrate est un médicament anti-tussif très utilisé, considéré comme non-toxique et qui remplace progressivement les médicaments opiacés.

Le Dextrométhorphane est utilisé essentiellement par voie orale sous forme de comprimés ou de sirops à des doses assez élevées (15 à 60 mg soit 0,25 à 1 mg/kg) en raison de la résorption relativement limitée de ce principe actif et de sa métabolisation en Dextrorphane inactif.

Cette métabolisation importante par voie orale permet de différencier trois types de population :
- 80 % de la population métabolise presque complètement et très rapidement le Dextrométhorphane et pour eux le Dextrométhorphane est inactif puisqu'on ne trouve pas de produit dans le sang.
- 13 % de la population ne métabolise pas le Dextrométhorphane et de ce fait on trouve des taux sanguins importants et persistants.
- et 6 % de la population métabolise lentement le Dextrométhorphane. On trouve de ce fait du Dextrométhorphane dont la demi-vie dans le plasma est de 12 heures et du Dextrorphane dont la demi-vie est de 6 heures.

Pour ces derniers sujets, l'élimination urinaire se fait pour une petite partie sous forme de Dextrométhorphane et en majorité sous forme de Dextrorphane.

Il est compréhensible que l'action anti-tussive du Dextrométhorphane administré par voie orale soit irrégulière ou fugace. Elle dépend essentiellement de la capacité de métabolisation de ce principe actif selon les sujets.

Les nouvelles compositions pour la voie transmuqueuse permettent de réaliser un mode d'administration très efficace, constant et rapide. Il est vraisemblable que le Dextrométhorphane administré sous cette forme traverse rapidement la barrière encéphalo-méningée et trouve, de ce fait, une efficacité plus grande sans métabolisation rapide ou préalable par la voie digestive et vraisemblablement dans le foie.

On peut en tirer la conclusion que sous cette forme le Dextrométhorphane est considérablement plus actif que par administration digestive tout en manifestant une durée d'action plus grande car le produit circule davantage dans le système sanguin.

L'invention consiste donc en ce qu'on administre par voie pernasale une préparation que contient à titre de principe actif du Dextrométhorphane ou un de ses sels d'addition avec un acide minéral ou organique en solution ou en suspension dans un excipient ou un véhicule inerte, non toxique, pharmaceutiquement acceptable associé ou non à un gaz propulseur, adapté à l'administration par voie pernasale.

Les compositions pharmaceutiques, selon l'invention, peuvent être préparées selon les méthodes connues de la pharmacotechnie. Ces compositions peuvent être des solutions aqueuses d'un sel de Dextrométhorphane, additionnées éventuellement d'un agent épaississant comme la méthylcellulose ou l'hydroxypropylméthyl cellulose ; d'un agent isotonisant comme un sel de sodium, de lithium, d'ammonium ou de calcium ou du sorbitol ; d'un agent tensio-actif, d'un agent bactéricide, d'un agent de conservation ou d'un agent polaire facilitant le passage à travers les muqueuses comme par exemple l'alcool benzylique ou le diméthylsulfoxyde.

De telles compositions liquides peuvent être présentées en nébuliseurs, en flacons compte-gouttes, en flacons pulvérisateurs souples ou en flacons-pompe.

Le Dextrométhorphane peut également être utilisé en suspension dans une solution aqueuse. On peut également utiliser une suspension ou une dispersion d'un sel de Dextrométhorphane peu soluble dans les milieux aqueux dans un véhicule approprié.

Les concentrations en principe actif peuvent varier de 0,2 mg à 2 mg pour 100 ml de préparation et les volumes administrés par un flacon pulvérisateur ou un flacon-pompe sont de l'ordre de 0,1 à 0,6 ml. L'administration unitaire est de ce fait comprise entre 0,2 µg et 12 µg et de préférence varie de 5 à 10 µg par prise.

L'administration se fait en pulvérisant la solution ou la suspension de Dextrométhorphane dans chaque narine une ou deux fois consécutives. Elle peut être renouvelée toutes les troix à six heures en fonction des besoins thérapeutiques.

## Revendications (Revendications pour l'(les) Etat(s) contractant(s) suivant(s): AT, BE, CH, DE, FR, GB, IT, LI, LU, NL, SE)

1. De nouvelles compositions pharmaceutiques anti-tussives destinées à la voie pernasale, qui renferment à titre de principe actif une quantité efficace de dextrométhorphane ou d'un de ses sels d'addition avec un acide minéral ou organique, en suspension, ou en solution dans un véhicule aqueux approprié pour l'administration permuqueuse caractérisé en ce que la concentration en principe actifs' échelonne de 0,2 à 2 mg pour 100 ml.

2. Une composition pharmaceutique selon la revendication 1 dans laquelle le principe actif est le bromhydrate de dextrométhorphane à une concentration de 0,2 à 2 mg pour 100 ml.

3. Une composition pharmaceutique selon l'une des revendications 1 et 2 dans laquelle la solution ou la suspension de dextrométhorphane est placée sous une pression de gaz supérieure à la pression atmosphérique.

4. Une composition pharmaceutique selon l'une des revendications 1 à 3 dans laquelle la solution ou la suspension de dextrométhorphane est additionnée d'un agent épaississant et/ ou d'un agent isotonisant et/ ou d'un agent tensioactife et/ ou d'un agent polaire.

5. Un procédé d'obtention des compositions pharmaceutiques selon l'une des revendications 1 à 4 dans laquelle on dissout ou met en suspension le dextrométhorphane ou un de ses sels d'addition avec un acide minéral ou organique avec un soluté aqueux et met, si désiré, la solution en résultant sous pression d'un gaz propulseur dans un récipient clos.

## Revendications (Revendications pour l'(les) Etat(s) contractant(s) suivant(s): ES, GR)

1. Procédé pour l'obtention d'un médicament anti-tussif efficace par voie pernasale caractérisé en ce qu'on incorpore le Dextrométhorphane ou un de ses sels d'addition avec un acide minéral ou organique en quantité s'échelonnant de 0,2 mg à 2 mg pour 100 ml avec un diluant ou véhicule inerte, non toxique, approprié pour l'administration par voie pernasale.

## Claims (Claims for the following Contracting State(s): AT, BE, CH, DE, FR, GB, IT, LI, LU, NL, SE)

1. Novel anti-cough pharmaceutical compositions intended for the pernasal way of administration which contain as active ingredient an efficient amount of Dextromethorphan or an acid addition with a mineral or organic acid, suspended or dissolved in an aqueous vehicle suitable for the permucous administration wherein the content in active principle ranges from .2 to 2 mg in 100 ml.

2. A pharmaceutical composition according to claim 1 wherein the active ingredient is Dextromethorphan as the hydrobromide at a concentration from .2 to 2 mg for 100 ml.

3. A pharmaceutical composition according to any of claims 1 and 2 wherein the solution or suspension of Dextromethorphan is put under a pressure of gas higher than the atmospheric pressure.

4. A pharmaceutical composition according to any of claims 1 to 3 wherein the solution or suspension of Dextromethorphan is added with a thickening agent and/or an isotonizing agent and/or a surfactant and/or a polar agent.

5. A process for producing the pharmaceutical compositions according to any of claims 1 to 4 wherein Dextromethorphan or an acid addition salt with a mineral or organic acid is dissolved or suspended in an aqueous solute and one placed, when desired, the resulting solution under the pressure of a propelling gas in a closed container.

## Claims (Claims for the following Contracting State(s): ES, GR)

1. Process for producing an anti-cough drug efficacious per pernasal way wherein Dextromethorphan or an acid addition salt thereof with a mineral or organic acid in an amount ranging from .2 to 2 mg per 100ml, is incorporated with an inert, non-toxic diluent or vehicle, suitable for administration through pernasal way of administration.

## Patentansprüche (Patentansprüche für folgende(n) Vertragsstaat(en): AT, BE, CH, DE, FR, GB, IT, LI, LU, NL, SE)

1. Neue pharmazeutische hüstenhemmende für den pernasalen Weg geeignete Zubereitungen, die als Wirkstoff, eine wirksame Menge an Dextromethorphan oder eine Salz davon mit einer anorganischen oder organischen Säure, suspendiert oder aufgelost in eine wässrige, für die permükale Verabreichung geeignete Vehikel, enthalten, dadurch gekennzeichnet dass die Konzentration an Wirkstoff von 0.2 bis 2 mg für 100 ml erstreckt.

2. Eine pharmazeutische Zubereitung nach Anspruch 1, worin der Wirkstoff Dextromethorphan Bromwasserstoff in einer Menge von 0.2 bis 2mg für 100 ml, ist.

3. Eine pharmazeutische Zubereitung nach einer der Ansprüche 1 und 2, worin die Lösung oder Suspension von Dextromethorphan unter eine Drück hoher als die atmosphärische Drück gestellt wird.

4. Eine pharmazeutische Zubereitung nach einer der Ansprüche 1 bis 3, worin die Lösung oder Suspension von Dextromethorphan mit einer Verdickungsmittel und/oder einer Isotonisierungs Mittel und/oder einem Tensid und/oder einem polarem Mittel, vermischt wird.

5. Verfahren zur Herstellung der pharmazeutischen Zubereitungen nach einer der Ansprüche 1 bis 4, worin Dextromethorphan oder eine seiner mit einer anorganischen oder organischen Säure Additions Salz, in einem wässrigen Medium aufgelöst oder supendiert wird, und gewünschenfalls die so gewonnene Lösung unter Drück eines vertreibenden Gas in einem verschlossenen Behälter, gestellt wird.

## Patentansprüche (Patentansprüche für folgende(n) Vertragsstaat(en): ES, GR)

1. Verfahren fur Herstellung eines husten-hemmenden, durch pernasalen Weg wirkenden Arzneimittel, dadurch gekennzeichnet dass Dextromethorphan order eine seiner mit einer anorganischen order organischen Säure Additions Salz in einer von 0.2 mg bis 2 mg per 100 mi erstreckende Menge, in einem inerten, ungiftigen, für die durch pernasalen Weg Verabreichung geeigneten Verdünnungsmittel or Vehikel eingefügt wird.
